# EUROPEAN PATENT APPLICATION

(11) **EP 2 340 808 A1**
(43) Date of publication of application: **06.07.2011**
(21) Application number: 09425517.1
(22) Date of filing: 21.12.2009
(51) Int. Cl.: A61K 9/00, A61K 9/08, A61K 9/10, A61K 9/20, A61K 9/28, A61K 9/48, A61K 31/196, A61K 36/185, A61K 36/53, A61P 1/04, A61K 36/80, A61K 36/74, A61K 36/68, A61K 36/64, A61K 36/634

(54) **Synergic combination of phenylpropanoids, such as verbascoside or teupolioside, and mesalamine**

(71) Applicant: I.R.B. Istituto Di Ricerche Biotecnologiche S.r.l., 36077 Altavilla Vicentina (VI) (IT)
(72) Inventor: Dal Toso, Roberto, 36077 Altavilla Vicentina, Vicenza (IT); Cuzzocrea, Salvatore, 36077 Altavilla Vicentina, Vicenza (IT); Pressi, Giovanna, 36077 Altavilla Vicentina, Vicenza (IT)
(74) Representative: Carangelo, Pierluigi

(57) **Abstract**

The present invention relates to the synergic combination of mesalamine with phenylpropanoids (verbascoside or teupolioside), obtained from plant cell cultures, and the pharmacological and nutritional use thereof for the prevention and treatment of inflammatory disorders of the digestive apparatus.

In particular, the present invention relates to a pharmaceutical composition containing mesalamine and one or more phenylpropanoids, together with pharmaceutically acceptable excipients.

## Description

### FIELD OF THE INVENTION

The present invention relates to the synergic combination of mesalamine with phenylpropanoids (verbascoside or teupolioside), obtained from plant cell cultures, and the pharmacological and nutritional use thereof for the prevention and treatment of inflammatory disorders of the digestive apparatus.

### STATE OF THE ART

Mesalamine, also known as 5-aminosalicilic acid (5-ASA), is an anti-inflammatory drug used extensively in the treatment of numerous disturbances of the gastrointestinal apparatus, including Crohn's disease and ulcerative colitis. Mesalamine is a drug that is metabolised in the intestine, the location where it performs its pharmacological activity.

Verbascoside and teupolioside are secondary metabolites of polyphenolic nature belonging to the glycosidic phenylpropanoids and found extensively in plants. The beneficial role played by glycosidic phenylpropanoids with regard to human health are well described in the literature. Said compounds are effective in preventing the onset of cancer (Kurkin V.A., Chem. Nat. Comp., 2003) and have anti-inflammatory (Lee J.Y., J. Ethnopharmacol, 2005; Korkina LG et al., 2007, Cellular and Molecular Biology 53(5): 78-83; Mazzon E. et al., 2009, Naunyn Schmiedebergs Arch. Pharmacol. 380(1): 79-94; Di Paola et al., 2009, Biochem. Pharmacol. 77 (5) : 845-57), anti-thrombotic (Tognolini M. et al., Life Sci., 2006), wound-healing (Hsu S., J. Am. Acad. Dermat., 2005) and cardioprotective activity (P.M. Etherton, Am. J. Med., 2002). The majority of said activities have been attributed to the chelating, antioxidant and radical scavenging properties exercised by said compounds. Glycosidic phenylpropanoids are present in very low quantities in the aerial parts of plants belonging to numerous plant species. This is the reason why the industrial and commercial development of said compounds has been very limited to date. The use of plant cell culture technology allows the attainment of highly selected cell lines with high glycosidic phenylpropanoid contents. In particular, as described in patents EP-A-1 736 166 and EP-A-1 736 167, cell lines with high teupolioside (*Ajuga reptans* cell line IRBN22-DSM16451) content and high verbascoside (*Syringa vulgaris* cell line IRB-SV25/B-DSM19511) content have been selected. Numerous literature citations indicate that free oxygen radicals, such as superoxides (02-), peroxynitrites (NO˙) and hydroxides (OH˙), play an important role in mediating the onset of the damage that occurs during inflammatory intestinal disorders. The intestine is well equipped with enzymes capable of producing free radicals (Parks, 1989). Furthermore, in the presence of inflammation, numerous phagocytic cells, capable of further increasing free radical levels, are attracted and activated. Several studies indicate that peripheral blood monocytes (Kitahora et al., 1988) and intestinal macrophages (Verspaget and Berken, 1985; Mahida et al., 1989), isolated from patients with intestinal inflammatory disorders, produce high quantities of free radicals. Furthermore, in such patients, it has been observed that high numbers of peripheral neutrophils (active free radical producers) migrate towards the intestinal wall (Crama-Bahbout et al., 1988). Grisham and Granger, 1988, have demonstrated that transient ischaemias and subsequent reperfusions, resulting in high free radical production, occur in ulcerative colitis. Ulcerative colitis is a process characterised by a cascade of events causing the recruitment and activation of polymorphonuclear leukocytes. The final result on completion of such events is ulceration of the intestinal mucosa. Wakefield et al., 1989, observed multifocal intestinal infarctions in patients affected by Crohn's disease, clearly indicating that localised ischaemia/reperfusion processes occur in such disorders. In the light of this data, the administration of molecules with high antioxidant and radical scavenging activity certainly plays an important role in preventing the onset of such disorders. Furthermore, it is known from the literature that molecules with antioxidant activity play a fundamental role in the prevention of vascular disorders and several inflammatory pathologies (Salvemini D., Cuzzocrea S., 2002, Free Radical Biology & Medicine, 33-9:1173-1185). Thanks to their antioxidant and radical scavenging activities, these molecules (particularly those belonging to the polyphenols) are capable of inhibiting the oxidation of low density lipoprotein (LDL) and inhibit platelet aggregation. Said compounds are capable of modulating the generation of nitric oxide (NO˙) produced by vascular endothelium and interfering with the mechanisms leading to inflammation and endothelial apoptosis, thus contributing towards preventing the endothelial malfunctions that play a crucial role in the pathogenesis of venous insufficiency and of numerous inflammatory conditions.

### SUMMARY OF THE INVENTION

It has been found that a phenylpropanoid in combination with mesalamine act synergically in the prevention and treatment of inflammatory conditions affecting the gastrointestinal tract.

Hence, a first object of the invention is a composition consisting of a phenylpropanoid, preferably verbascoside or teupolioside, and mesalamine.

A second object of the invention relates to the use of the composition consisting of a phenylpropanoid and mesalamine in the preparation of nutritional and pharmaceutical products for human or veterinary use in the prevention and in the treatment of inflammatory conditions affecting the gastrointestinal tract.

Further objects, characteristics and the advantages of the present invention will become clearer from the following detailed description.

### Brief description of the figures

**Figure 1** shows the reduction in histological damage from DNBS-induced colitis following oral administration of the synergic combination of verbascoside (Ver 0.1 mg/Kg) and mesalamine (Mes 100 mg/Kg).
**Figure 2** shows the reduction in myeloperoxidase activity following oral administration of the synergic combination of verbascoside (Ver 0.1 mg/Kg) and mesalamine (Mes 100 mg/Kg).
**Figure 3** shows the reduction in weight loss following oral administration of the synergic combination of verbascoside (Ver 0.1 mg/Kg) and mesalamine (Mes 100 mg/Kg).
**Figure 4** shows the reduction in the release of the cytokine TNF-alpha following oral administration of the synergic combination of verbascoside (Ver 0.1 mg/Kg) and mesalamine (Mes 100 mg/Kg).
**Figure 5** shows the reduction in the release of the cytokine IL-1β following oral administration of the synergic combination of verbascoside (Ver 0.1 mg/Kg) and mesalamine (Mes 100 mg/Kg).
**Figure 6** shows the reduction in histological damage from DNBS-induced colitis following oral administration of the synergic combination of Teupolioside (Teu 0.1 mg/Kg) and mesalamine (Mes 100 mg/Kg).
**Figure 7** shows the reduction in myeloperoxidase activity following oral administration of the synergic combination of Teupolioside (Teu 0.1 mg/Kg) and mesalamine (Mes 100 mg/Kg).
**Figure 8** shows the reduction in weight loss following oral administration of the synergic combination of Teupolioside (Teu 0.1 mg/Kg) and mesalamine (Mes 100 mg/Kg).
**Figure 9** shows the reduction in the release of the cytokine TNF-alpha following oral administration of the synergic combination of teupolioside (Teu 0.1 mg/Kg) and mesalamine (Mes 100 mg/Kg).
**Figure 10** shows the reduction in the release of the cytokine IL-1β following oral administration of the synergic combination of teupolioside (Teu 0.1 mg/Kg) and mesalamine (Mes 100 mg/Kg).

### DETAILED DESCRIPTION OF THE INVENTION

Surprisingly, a synergic anti-inflammatory effect has been observed with a phenylpropanoid (verbascoside or teupolioside) in conjunction with mesalamine in a DNBS-induced colitis model. The verbascoside used has been extracted from cultures of the Syringa vulgaris IRB-SV25/B DSMZ selected cell line (Deutsche Sammlung von Mikroorganismen und Zellkulturen, Braunschweig, D) accession No.: DSM 16857, accession date: 2004-09-15) as described in patent EP-A-1 736 167. The teupolioside has been extracted from cultures of the Ajuga reptans IRB-N22 DSMZ selected cell line (Deutshe Sammlung von Mikroorganismen und Zellkulturen, Braunschweig, D): DSM 16451, accession date: 31-03 2004) as described in patent EP-A-1 736 166.

The anti-inflammatory activity of the phenylpropanoid (verbascoside or teupolioside) and mesalamine synergic combination are described in example 1 and example 2.

### EXAMPLE 1

Evaluation of the anti-inflammatory activity of the verbascoside and mesalamine combination has been determined in an in vivo induced colitis model. Colitis has been induced in rats using a colon inflammation model, stimulated by DNBS (2,4,6 dinitrobenzenesulphonic acid). After 4 days of induction of inflammation, the following parameters have been evaluated: determination of colon damage by means of morphological and histological analysis; determination of myeloperoxidase activity, determination of body weight, determination of TNF-alpha and IL 1-β.

Animals have been subdivided into 5 test groups (10 Sprague-Dawley rats weighing 200-250 g in each group):
1. **one group to which only carrier has been** administered(carrier);
2. **one group to which DNBS has been** administered(DNBS+carrier);
3.one group to which DNBS and 0.1 mg/Kg verbascoside has been administered; the verbascoside has been administered orally, at a daily concentration of 0.1 mg/Kg, for 4 days after the administration of DNBS;
4. one group to which DNBS and 100 mg/Kg mesalamine have been administered; the mesalamine has been administered orally, at a daily concentration of 100 mg/Kg, for 4 days after the administration of DNBS;
5. one group to which DNBS, 100 mg/Kg mesalamine and 0.1 mg/Kg of verbascoside have been administered; the mesalamine and verbascoside combination has been administered orally, at a daily concentration of 100 mg/Kg, for 4 days after the administration of DNBS. Four days after the administration of DNBS, the following investigations have been conducted:
   - evaluation of colon damage by means of morphological and histological analysis (a score is assigned on the basis of the damage observed);
   - evaluation of myeloperoxidase activity
   - determination of body weight and
   - determination of the levels of the cytokines TNF-alpha and IL 1-β

### Evaluation of colon damage

Evaluation of colon damage has been determined by assigning scores based on the damage detected by macroscopic and histological analysis. The scores assigned have been as follows:
0: no damage;
1: slight damage, with focal epithelial damage and necrosis;
2: moderate damage, necrosis of the villi and widespread oedema;
3: severe damage, necrosis with the presence of neutrophil infiltration;
4: very severe damage, widespread necrosis with massive neutrophil infiltration and haemorrhaging.

### Determination of myeloperoxidase activity

Myeloperoxidase activity is an indicator of polymorphonuclear leukocyte accumulation. Myeloperoxidase activity has been evaluated on portions of colon tissue 4 days after DNBS administration. The tissue portions have been homogenised in a solution of 10 mM potassium phosphate buffer (pH 7.0) containing 0.5% hexadecyl trimethyl ammonium bromide. The homogenates have been centrifuged at 20000 X g for 30 minutes at 4°C. An aliquot of supernatant has been used to react with tetramethyl benzidine (1 . 6 mM) and 0.1 mM hydrogen peroxide. Absorbance has been measured at 650 nm. MPO activity is defined as the quantity of enzyme capable of degrading 1 µmole of peroxide per minute at 37°C and is expressed as U/g of fresh weight of tissue.

### Determination of cytokine content

Cytokine content has been determined in colon tissues collected 4 days after DNBS administration. Portions of terminal colon have been homogenised in PBS containing 2 mM phenylmethyl sulphonyl fluoride. The assay has been conducted using a commercially available colorimetric kit (Calbiochem-Novobiochem Corporation, USA) used in accordance with the manufacturer's instructions. All determinations have been performed in duplicate on serial dilutions.

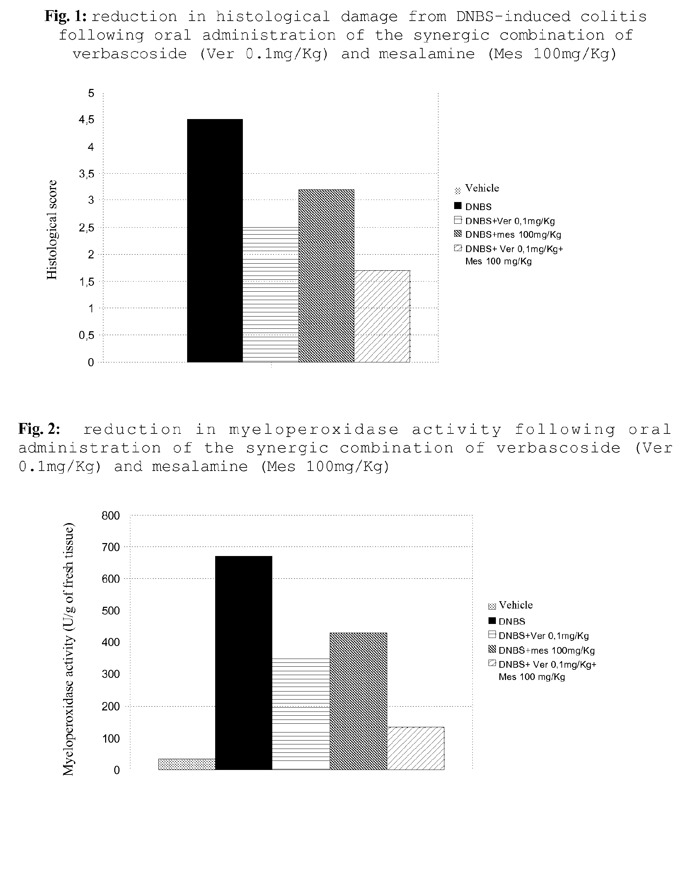

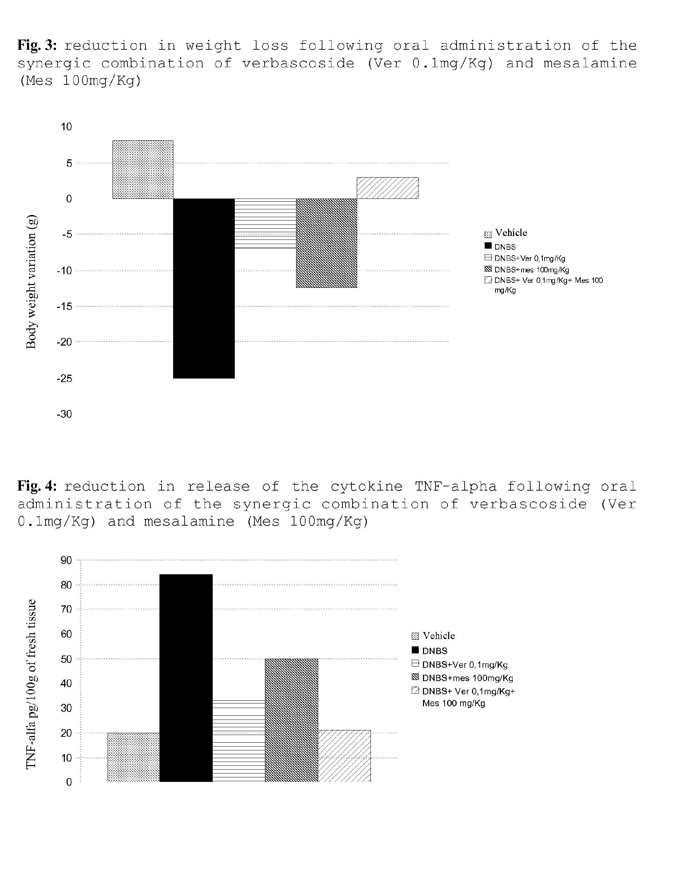

The results obtained are summarised in the graphs shown in fig. 1, fig. 2, fig. 3, fig. 4 and fig. 5.

With respect to verbascoside and mesalamine administered individually, treatment involving oral administration of the verbascoside and mesalamine combination significantly and synergistically reduces:
- the extent and severity of histological colon damage;
- the degree of polymorphonuclear leukocyte infiltration;
- the loss of body weight with respect to controls;
- release of the cytokines TNF-alpha and IL 1-□.

### EXAMPLE 2

Evaluation of the anti-inflammatory activity of the teupolioside and mesalamine combination has been determined in an in vivo induced colitis model, using the same method described in example 1.

The animals used in testing have been subdivided into 5 test groups (10 Sprague-Dawley rats weighing 200-250 g in each group):
1.one group to which only carrier has been administered (carrier);
2.one group to which DNBS has been administered (DNBS+carrier);
3.one group to which DNBS and 0.1 mg/Kg teupolioside have been administered; the teupolioside has been administered orally, at a daily concentration of 0.1 mg/Kg, for 4 days after the administration of DNBS;
4. one group to which DNBS and 100 mg/Kg mesalamine have been administered; the mesalamine has been administered orally, at a daily concentration of 100 mg/Kg, for 4 days after the administration of DNBS;
5. one group to which DNBS, 100 mg/Kg mesalamine and 0.1 mg/Kg of teupolioside have been administered; the mesalamine and teupolioside combination has been administered orally, at a daily concentration of 100 mg/Kg, for 4 days after the administration of DNBS.

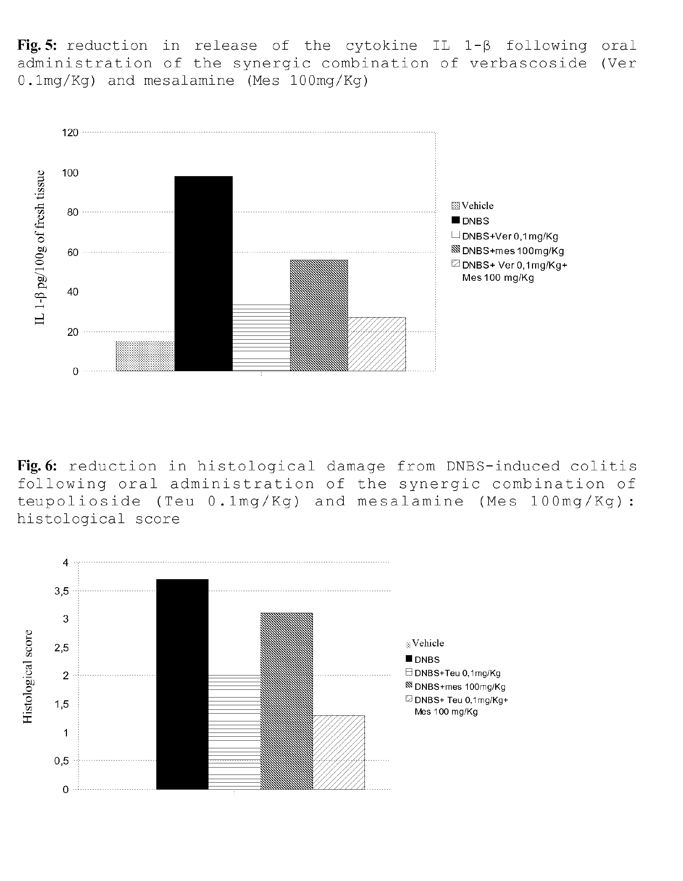

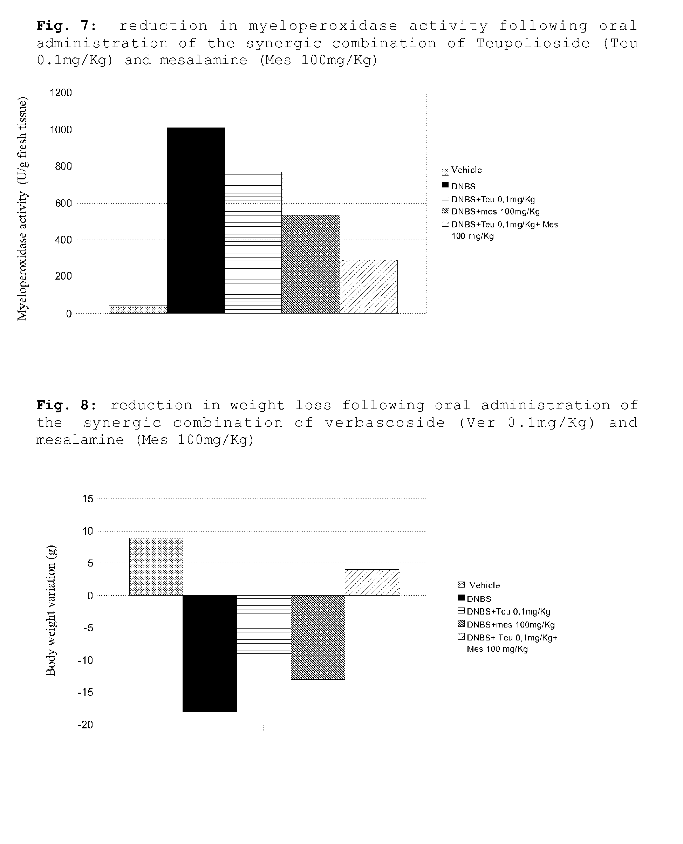

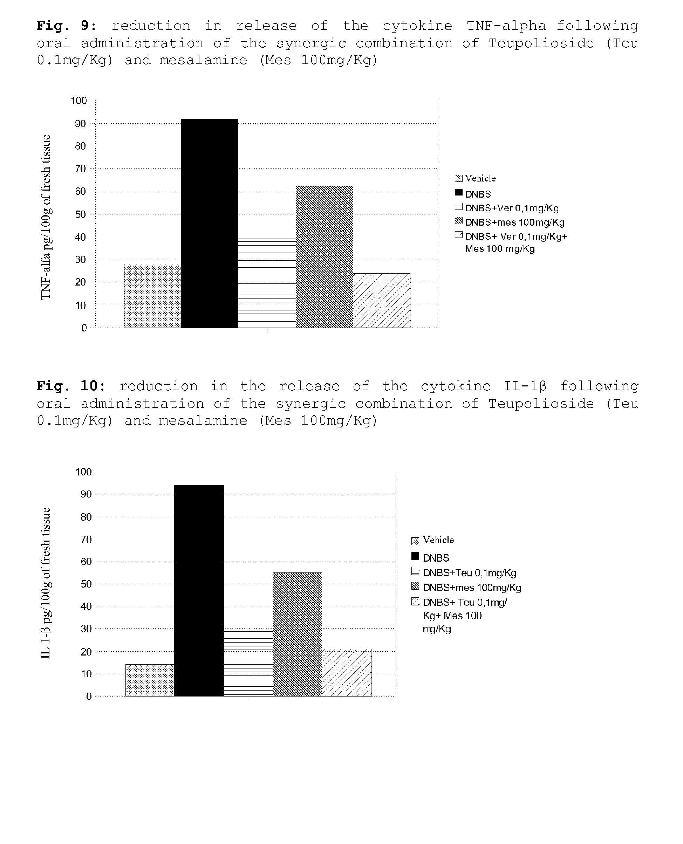

The results obtained are summarised in the graphs shown in fig. 6, fig. 7, fig. 8, fig. 9 and fig. 10.

With respect to teupolioside and mesalamine administered individually, treatment involving oral administration of the teupolioside and mesalamine combination significantly and synergistically reduces:
- the extent and severity of histological colon damage;
- the degree of infiltration of polymorphonuclear leukocytes and the reduction in body weight;
- release of the cytokines TNF-alpha and IL 1-β.

With regard to their biological activities (as described in examples 1 and 2), the phenylpropanoid and mesalamine combinations forming the object of the present invention are valid tools to be used for the prevention and treatment of inflammatory conditions affecting the gastrointestinal tract, such as ulcerative colitis, irritable bowel syndrome and Crohn's disease. The aforementioned synergic combinations may be validly used in the preparation of nutritional and pharmaceutical formulations for both human and veterinary use.

The quantity and dosage of the combinations in compositions for nutritional and pharmaceutical use is determined considering the various factors including the scope of administration, the patient's age, sex and body weight.

One or more phenylpropanoids in combination with mesalamine is envisaged. The phenylpropanoids are preferably selected from verbascoside, teupolioside, isoverbascoside, isoteupolioside, methylteuplioside, echinacoside, campneoside II, forsythoside, plantamajoside, leucosceptoside A, martynoside, plantainoside, 3 , 4-dicaffeoylquinic acid, 3,5-dicaffeoylquinic acid, 1,5-dicaffeoylquinic acid, 1,4-dicaffeoylquinic acid, 3,5-dicaffeoylquinic acid, 4-malonylquinic acid, chlorogenic acid, more preferably verbascoside and teupolioside.

The prevention and treatment method according to the invention may consist of the simultaneous, sequential or separate administration of the phenylpropanoids and mesalamine.

An appropriate dosage range for the combination should be approximately between 0.4 g and 4.8 g/day of mesalamine and between 0.1 mg and 100 mg/day of verbascoside and/or teupolioside.

The composition should be administered orally or parenterally for systemic administration and rectally for local administration.

In the case of simultaneous administration of the active substances, they can be formulated in a single nutritional of pharmaceutical composition, together with nutritionally or pharmaceutically acceptable excipients, as appropriate. Said composition preferably contains mesalamine and phenylpropanoids in weight ratios varying between 4,000:1 and 4:1, preferably between 1,500:1 and 500:1, more preferably between 1,100:1 and 900:1.

The composition containing phenylpropanoids and mesalamine may be prepared in accordance with conventional methods, such as those described in Remington's Pharmaceutical Sciences Handbook, Mack Pub. Co., N.Y., USA, 17th edition, 1985.

The composition according to the present invention may preferably be formulated for oral, parenteral or local rectal administration.

Potential forms of administration are described hereinafter.

For oral administration, the pharmaceutical compositions may be, for example, in the form of tablets or capsules, prepared in the conventional manner with the aid of pharmaceutically acceptable excipients such as binding agents (for example pre-gelatinised corn starch, polyvinylpyrrolidone or hydroxypropyl methylcellulose); fillers (for example lactose, microcrystalline cellulose or calcium hydrogen phosphate); lubricants (for example magnesium stearate, talc or silica); disintegrants (for example potato starch or sodium starch glycolate); or inhibiting agents (for example sodium lauryl sulphate).

Tablets may be coated, using methods well known in the art. Liquid preparations for oral administration may be, for example, in the form of solutions, syrups or suspensions or may be in the form of lyophilised products to be reconstituted with water or other suitable carriers prior to use. Such liquid preparations may be prepared using conventional methods with pharmaceutically acceptable additives such as suspension agents (for example sorbitol syrup, cellulose derivatives or hydrogenated edible fats); emulsifiers (for example lecithin or acacia); non-aqueous carriers (for example almond oil, oily esters, ethyl alcohol or fractionated vegetable oils); and preservatives (for example methyl-or propyl-p-hydroxybenzoates or sorbic acid). The preparation may also appropriately contain flavourings, colourants and sweeteners.

Preparations for oral administration may be appropriately formulated to allow the controlled release of the active substance.

For buccal administration, the compositions may be in the form of conventionally formulated tablets or lozenges, adapted for absorption by the buccal mucosa. Typical buccal formulations include tablets for sublingual administration.

The compounds according to the present invention may be formulated for parenteral administration by injection. Formulations for injections may be presented in single dose form, for example in vials, with added preservative. The compositions may be delivered in this form as suspensions, solutions or emulsions in oily or aqueous carriers and may contain formulary agents such as suspension agents, stabilisers and/or dispersants. Alternatively, the active ingredient may be in powder form for reconstitution using an appropriate carrier, for example sterile water, prior to use.

According to the present invention, the compounds may also be formulated in accordance with rectal compositions such as suppositories or retention enemas, for example containing common basic suppository components such as cocoa butter or other glycerides.

In addition to the previously described compositions, the compounds may also be formulated as deposit preparations. Such long acting preparations may be administered by implantation (for example subcutaneously, transcutaneously or intramuscularly) or by intramuscular injection. Thus, for example, the compounds according to the present invention may be formulated with appropriate polymeric or hydrophobic materials (for example in the form of an emulsion in a suitable oil) or ion exchange resin or as sparingly soluble derivatives, for example as a sparingly soluble salt.

## Claims

1. A pharmaceutical composition containing mesalamine and one or more phenylpropanoids, together with pharmaceutically acceptable excipients.

2. The composition according to claim 1, wherein said one or more phenylpropanoids are selected from verbascoside, teupolioside, isoverbascoside, isoteupolioside, methylteuplioside, echinacoside, campneoside II, forsythoside, plantamajoside, leucosceptoside A, martynoside, plantainoside, 3,4-dicaffeoylquinic acid, 3,5-dicaffeoylquinic acid, 1,5-dicaffeoylquinic acid, 1,4-dicaffeoylquinic acid, 3,5-dicaffeoylquinic acid, 4-malonylquinic acid, chlorogenic acid.

3. The composition according to claim 2, wherein said one or more phenylpropanoids are verbascoside and/or teupolioside.

4. The composition according to any of the claims 1 to 3, wherein said mesalamine and said one or more phenylpropanoids are present in a weight ratio of between 4,000:1 and 4:1.

5. The composition according to claim 4, wherein said mesalamine and said one or more phenylpropanoids are present in a weight ratio of between 1,500:1 and 500:1.

6. The composition according to claim 4 or 5, wherein said mesalamine and said one or more phenylpropanoids are present in a weight ratio of between 1,100:1 and 900:1.

7. The composition according to any of the claims 1 to 6, wherein said composition is adapted for oral or parenteral systemic administration or rectal local administration.

8. Mesalamine for use, in association with one or more phenylpropanoids, in the prevention and treatment of inflammatory conditions affecting the gastrointestinal tract.

9. Mesalamine for use in association with one or more phenylpropanoids, according to claim 8, in the prevention and treatment of ulcerative colitis, irritable bowel syndrome and/or Crohn's disease.

10. Mesalamine for use in association with one or more phenylpropanoids, according to claim 8 or 9, wherein said phenylpropanoids are verbascoside and/or teupolioside.

11. Mesalamine for use in association with one or more phenylpropanoids, according to any of the claims 8 to 10, wherein said use envisages the preparation of a medication wherein said mesalamine and said one or more phenylpropanoids are present in combined form or separately.

12. Mesalamine for use in association with one or more phenylpropanoids according to any of the claims 8 to 11, wherein said use envisages the administration of mesalamine in quantities of between 0.4 and 4.8 g per day and the administration of said one or more phenylpropanoids in quantities of between 0.1 and 100 mg per day.

13. Mesalamine for use in association with one or more phenylpropanoids, according to any of the claims 8 to 12, wherein said mesalamine and said one or more phenylpropanoids are used in weight ratios of between 4,000:1 and 4:1, or between 1,500:1 and 500:1, or between 1,100:1 and 900:1.

14. A nutritional composition containing mesalamine and one or more phenylpropanoids, as defined in any of the claims 2 to 6.
